# EUROPÄISCHE PATENTSCHRIFT

(11) **EP 1 275 639 B1**
(45) Veröffentlichungstag und Bekanntmachung des Hinweises auf die Patenterteilung: **07.06.2006**
(21) Anmeldenummer: 02014038.0
(22) Anmeldetag: 28.06.2002
(51) Int. Cl.: C07C 263/10

(54) **Verfahren zur Herstellung von (cyclo)aliphatischen Isocyanaten**
Process for the preparation of (cyclo)aliphatic isocyanates
Procédé de préparation de composés isocyanates (cyclo)aliphatique

(30) Priorität: 11.07.2001 DE 10133729
(43) Veröffentlichungstag der Anmeldung: 15.01.2003
(73) Patentinhaber: Bayer MaterialScience AG, 51368 Leverkusen (DE)
(72) Erfinder: Leimkühler, Hans-Joachim, Dr., 51375 Leverkusen (DE); Stutz, Herbert, Dr., 41541 Dormagen (DE); Leuckel, Wolfgang, Prof. Dr., 67098 Bad Dürkheim (DE)

(56) Entgegenhaltungen:
- EP-A- 0 322 647
- EP-A- 0 593 334
- EP-A- 0 676 393
- EP-A- 0 749 958
- EP-A- 0 928 785
- US-A- 4 847 408

## Beschreibung

Die vorliegende Erfindung betrifft ein neues Verfahren zur Herstellung von (cyclo)aliphatischen Diisocyanaten und Triisocyanaten durch Phosgenierung von (cyclo)aliphatischen Diaminen und Triaminen in der Gasphase unter Einsatz spezieller Reaktoren.

Es ist bereits bekannt, dass (cyclo)aliphatische Diamine in der Gasphase phosgeniert werden können. In SU-A 00 407 567 wird dazu ein von außen und innen beheizter Reaktor benutzt, der mit Kühlschlangen versehen ist, um die Reaktionswärme abzuführen. Dieser Reaktor hat auf der Innenseite einen Halter für Aggregate zum Vorheizen, um den herum Rohre angeordnet sind, durch die die Reaktanden zugeführt werden. Der Reaktor selbst verfügt außerdem über externe Erhitzer. Die Vorheizer und die externen Erhitzer werden angeschaltet, bis die Temperatur 210°C bis 230°C erreicht. Eine stöchiometrische Menge Phosgen wird danach durch ein Rohr zugeführt, während das gasförmige oder flüssige Amin durch das andere Rohr den Reaktor erreicht. Letzteres wurde zuvor auf eine Temperatur von 30°C bis 60°C oberhalb der Siedetemperatur des Amins erhitzt. Gasförmiges Isocyanat bildet sich nun im Reaktor. Die Reaktionswärme wird durch Kühlschlangen abgeführt. Wenn diese ausreichend warm geworden sind, wird der Erhitzer abgeschaltet. Der externe Erhitzer wird nur angeschaltet, wenn es erforderlich ist, um Wärmeverluste auszugleichen, so dass das Produkt nicht unter 210°C bis 230°C abkühlt. Die gasförmigen Produkte verlassen den Reaktor durch einen Auslass. Nachteil dieses Reaktors ist, dass er nur zum batchweisen Betrieb geeignet ist.

In der GB-A 1 165 831 wird die Reaktion in einem mit einem mechanischen Rührer ausgestatteten Rohrreaktor durchgeführt. Der Reaktor ähnelt einem Dünnschichtverdampfer, bei dem der Rührer die Gase mischt und gleichzeitig die beheizten Wände des Rohrreaktors bestreicht, um so einen Aufbau von polymerem Material an der Rohrwand zu verhindern. Die Verwendung eines schnelllaufenden Rührers beim Umgang mit ca. 300°C heißem Phosgen erfordert jedoch hohen sicherheitstechnischen Aufwand, um den Reaktor abzudichten und den Rührer in dem hochkorrosiven Medium zu lagern.

In der EP-A 0 289 840 und in der EP-A 0 749 958 ist ein zylindrischer Reaktionsraum ohne sich bewegende Teile genannt, in dem man die Reaktanden unter Aufrechterhaltung einer turbulenten Strömung miteinander zur Reaktion bringt. Aufgrund der geometrischen Form des zylindrischen Reaktionsraums kommt es zu Rückvermischungsvorgängen, in folge derer die Produkte unter Entstehung von festen Ablagerungen mit dem Diamin aus dem Edukt reagieren. Dadurch kommt es zur Verschmutzung des Reaktors und zu Verstopfungen im Gasweg.

In der EP-A 0 593 334 wird die Phosgenierung aromatischer Diamine beschrieben. Dort werden die Reaktanden zunächst in einer turbulenten Strömung gemischt (bei Reynoldszahlen von mindestens 3000, besonders bevorzugt mindestens 8000). Anschließend wird die Reaktion bei laminaren oder turbulenten Strömungsbedingungen in einem Rohrreaktor ohne bewegliche Mischelemente und einer Einengung der Wände durchgeführt.

Die in der letztgenannten Anmeldung beschriebenen Erkenntnisse können nicht ohne weiteres von aromatischen Diaminen auf aliphatische oder cycloaliphatische Diamine oder Triamine übertragen werden, da sich die entsprechenden Reaktionen hinsichtlich Mechanismus, feststoffbildenden Nebenreaktionen und erforderlichen Reaktionszeiten fundamental voneinander unterscheiden.

In EP-A 676 392 wird ein Verfahren zur Gasphasenphosgenierung spezieller Diamine beschrieben, wobei Phosgen mit dampfförmigen Diaminen in einem zylindrischen Reaktor ohne bewegliche Teile unter Auttechterhaltung einer turbulenten Strömung zur Reaktion gebracht werden. Dieses Verfahren zeichnet sich durch eine verringerten Anteil an Nebenprodukten aus. Über Einbauten in den Eduktzuleitungen wird jedoch nichts ausgesagt.

Es wurde nun überraschenderweise gefunden, dass es möglich ist, (cyclo)aliphatische Isocyanate durch Gasphasenphosgenierung der ihnen zugrunde liegenden Amine unter Umgehung der genannten Nachteile des Standes der Technik herzustellen, wenn man die Reaktion in einem Reaktor durchführt, der in spezieller, nachstehend erläuterten Weise von der beschriebenen zylindrischen Form abweicht.

Verfahren zur Herstellung von (cyclo)aliphatischen Diisocyanaten und Triisocyanaten der allgemeinen Formel (I)

R-(NCO)ₙ (I),

in welcher
- R: für einen (cyclo)aliphatischen Kohlenwasserstoffrest mit bis zu 15 Kohlenstoffatomen und
- n: für die Zahl 2 oder 3 steht,
durch Phosgenierung der entsprechenden Diamine oder Triamine der allgemeinen Formel (II)

R-(NH₂)ₙ (II)

in welcher
- R und n: die bei Formel (I) angegebene Bedeutung haben,
in der Gasphase, wobei die dampfförmigen (cyclo)aliphatischen Amine, gegebenenfalls verdünnt mit einem Inertgas oder mit den Dämpfen eines inerten Lösungsmittels, und Phosgen getrennt auf Temperaturen von 200°C bis 600°C erhitzt, und kontinuierlich in einen Reaktionsraum ohne sich bewegende Teile mit Einengungen der Wände im Bereich der Reaktionszone zur Reaktion gebracht werden, dadurch gekennzeichnet, dass ein Reaktor verwendet wird, dessen Querschnittsfläche sich im Winkel von 80 bis 90° erweitert und bei dem das Verhältnis der Querschnittsflächen des Reaktionsraums nach und vor der Erweiterung bei 3:1 bis 7:1 liegt und wobei die Eduktströme kurz vor der Mischung im Reaktor über drallerzeugende Einbauten geleitet werden.

Ausgangsmaterialien für das erfindungsgemäße Verfahren sind (cyclo)aliphatische Diamine und Triamine der allgemeinen Formel (II)

R-(NH₂)ₙ (II),

in welcher
- R: für einen (cyclo)aliphatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
- n: für die Zahl 2 oder 3 steht.

Typische Beispiele geeigneter (cyclo-)aliphatischer Diamine sind 1,4-Diaminobutan, 1,6-Diaminohexan, 1,11-Diaminoundecan, 1,4-Diaminocyclohexan, 1-Amino-3,3,5-trimethyl-5-aminomethylcyclohexan (IPDA), 4,4'-Diaminodicyclohexylmethan oder 4,4'-Diaminodicyclohexylpropan-(2,2). Ein Beispiel für ein geeignetes (cyclo)aliphatisches Triamin ist 1,8-diamino-4-(aminomethyl)octan, Triaminononan. Bevorzugte Ausgangsamine sind 1,6-Diaminohexan, IPDA, 4,4'-Diaminodicyclohexylmethan und Triaminononan.

Erfindungsgemäß werden (cyclo)aliphatische Diisocyanate oder (cyclo)aliphatische Triisocyanate der Formel (I)

R-(NCO)ₙ (I),

in welcher
- R: für einen (cyclo)aliphatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind, und
- n: für die Zahl 2 oder 3 steht,
hergestellt.

Bevorzugt werden im erfmdungsgemäßen Verfahren 1,6-Diisocyanatohexan, 1-Isocyanato-3,3,5-trimethyl-5-isocyanatomethylcyclohexan (IPDI) oder 1,8-Diisocyanato-4-(isocyanatomethyl)octan hergestellt.

Die Ausgangsamine werden vor der Durchführung des erfindungsgemäßen Verfahrens verdampft und auf 200°C bis 600°C, vorzugsweise 250°C bis 500°C erhitzt und gegebenenfalls verdünnt mit einem Inertgas (z.B. N₂, Edelgas) und/oder mit den Dämpfen eines inerten Lösungsmittels (z.B. Monochlorbenzol oder Dichlorbenzol) dem Reaktor zugeführt.

Das bei der Phosgenierung verwendete Phosgen wird vor Durchführung des erfindungsgemäßen Verfahrens auf eine Temperatur innerhalb des Bereichs von 200°C bis 600°C, vorzugsweise 250°C bis 500°C erhitzt.

Zur Durchführung der erfindungsgemäßen Verfahrens werden die vorerhitzten und drallbehafteten Ströme von Amin bzw. Amin-Inertgas-Gemisch einerseits und von Phosgen andererseits kontinuierlich in einen Reaktionsraum ohne sich bewegende Teile mit Einengungen der Wände geleitet und dort miteinander vermischt.

Die absolute Größe des eingesetzten Apparates richtet sich dabei nach der herzustellenden Produktmenge.

Die Reaktionsräume weisen in Strömungsrichtung hinter der Vermischung der beiden Edukte eine schlagartige Erweiterung der Querschnittsfläche im Winkel von 80 bis 90°, bevorzugt 90° auf. Dabei liegt das Verhältnis der Querschnittsflächen des Reaktionsraums nach und vor der Erweiterung bei 3:1 bis 7:1, bevorzugt bei 4,5:1 bis 5,5:1. An dieser Erweiterungsstelle können sich Wirbel bilden, die die Hauptströmung von der Reaktorwand fernhalten. Diese Form kann somit das Anlegen der Reaktionszone an die Behälterwand reduzieren. Damit werden die polymerisationsfähigen Produkte von der Reaktorwand fern gehalten, was die Entstehung von festen Niederschlägen und damit mögliche Verschmutzungen reduziert.

Die Reaktoren bestehen im allgemeinen aus Stahl, Glas, legiertem oder emaillierten Stahl und weisen eine Länge auf, die ausreichend ist, um unter den Verfahrensbedingungen eine vollständige Umsetzung des Amins mit dem Phosgen zu ermöglichen. Die Gasströme werden im allgemeinen an einem Ende des Reaktionsraumes in diesen eingeleitet, wobei diese Einleitung beispielsweise durch an einem Ende des Reaktors angebrachte Düsen oder durch eine Kombination aus Düse und einem Ringspalt zwischen Düse und Wand erfolgen kann. Die Mischzone wird ebenfalls bei einer Temperatur innerhalb des Bereiches von 200°C und 600°C, vorzugsweise 250°C und 500°C gehalten, wobei diese Temperatur gegebenenfalls durch Beheizung des Reaktors aufrecht erhalten wird.

Bei der Durchführung des erfindungsgemäßen Verfahrens liegt im allgemeinen der Druck in den Zuleitungen zum Reaktionsraum bei 200 mbar bis 3000 mbar und am Ausgang des Reaktionsraumes bei 150 mbar bis 2000 mbar, wobei durch Aufrechterhaltung eines geeigneten Differenzdruckes eine Strömungsgeschwindigkeit innerhalb des Reaktionsraumes von mindestens 3 m/s, vorzugsweise mindestens 6 m/s und besonders bevorzugt 10 m/s bis 120 m/s Sorge getragen wird. Unter diesen Voraussetzungen herrschen innerhalb des Reaktionsraumes im allgemeinen turbulente Strömungsverhältnisse vor.

Es ist ein Vorteil des erfindungsgemäßen Verfahrens, dass bei gleicher oder besserer Produktqualität eine höhere Raum-Zeit-Ausbeute des Reaktors erreicht wird, indem die Standzeit des Reaktors, d.h. die Produktionszeit im Verhältnis zu Stillstandszeiten, die zur Reinigung des Reaktors erforderlich sind, je nach hergestelltem Isocyanat um 40-60 % erhöht werden kann.

### Beispiel

Das erfindungsgemäße Verfahren wird durch nachfolgendes Beispiel näher erläutert:

In ein Mischrohr mit nachgeschalteter Diisocyanatkondensationsstufe und dieser nachfolgende, mit Aktivkohle gefülltem Phosgen-Adsorptionsturm strömen kontinuierlich durch eine Düse, die in das Mischrohr hineinragt, 5,91 mol/h Phosgen, die in einem vorgeschalteten Wärmetauscher bei einem Druck von 1100 mbar auf eine Temperatur von 400°C erwärmt wurden. Durch den Ringspalt zwischen Düse und Mischrohr wird gleichzeitig ein auf 400°C erwärmtes Gemisch aus 1,26 mol gasförmigem Hexamethylendiamin und 1,25 mol Stickstoff stündlich in das Mischrohr geleitet. Der Durchmesser des Mischrohres variiert kaskadenförmig entlang der Längsachse, in dem er sich auf den ersten 1,5 mm ab Düse mit einem Winkel von 10° bis auf 2,5 mm verringert, anschließend auf den nächsten 17,5 mm konstant bleibt und sich in einem dritten Segment innerhalb von 20 mm Länge mit einem Winkel von zunächst 5° bis auf 6 mm erweitert (siehe Skizze des Reaktors). Durch Anlegen eines Vakuums am Ausgang aus dem Phosgenadsorptionsturm wird eine Druck im Mischrohr von ca. 350 mbar aufrechterhalten. Das heiße, den Reaktionsraum verlassene Reaktionsgemisch wird in einer Kondensationsstufe durch Dichlorbenzol geleitet, welches bei einer Temperatur von 150 - 160°C gehalten wird. Hierbei erfolgt eine selektive Kondensation des gebildeten Diisocyanatohexans. Das die Waschstufe durchlaufende, im wesentlichen aus Stickstoff, Chlorwasserstoff und überschüssigem Phosgen bestehende Gasgemisch wird in dem Adsorptionsturm anschließend vom Phosgen befreit. Das Diisocyanat wird aus dem Waschlösungsmittel destillativ in reiner Form gewonnen. Die Ausbeute an 1,6-Diisocyanatohexan liegt bei 98,0% der Theorie.

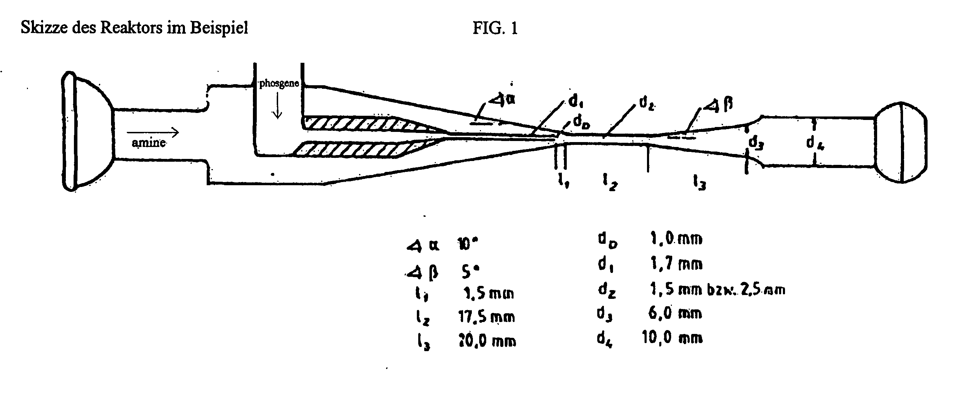

## Patentansprüche

1. Verfahren zur Herstellung von (cyclo)aliphatischen Diisocyanaten und Triisocyanaten der allgemeinen Formel (I)
R-(NCO)ₙ (I),
in welcher
R für einen (cyclo)aliphatischen Kohlenwasserstoffrest mit bis zu 15 Kohlenstoffatomen und
n für die Zahl 2 oder 3 steht,
durch Phosgenierung der entsprechenden Diamine oder Triamine der allgemeinen Formel (II)
R-(NH₂)ₙ (II),
in welcher
R und n die bei Formel (I) angegebene Bedeutung haben,
in der Gasphase wobei die dampfförmigen (cyclo)aliphatischen Amine, gegebenenfalls verdünnt mit einem Inertgas oder mit den Dämpfen eines inerten Lösungsmittels, und Phosgen getrennt auf Temperaturen von 200°C bis 600°C erhitzt, und kontinuierlich in einen Reaktionsraum ohne sich bewegende Teile mit Einengungen der Wände im Bereich der Reaktionszone zur Reaktion gebracht werden, **dadurch gekennzeichnet, dass** ein Reaktor verwendet wird, dessen Querschnittsfläche sich im Winkel von 80 bis 90° erweitert und bei dem das Verhältnis der Querschnittsflächen des Reaktionsraums nach und vor der Erweiterung bei 3:1 bis 7:1 liegt, und wobei die Eduktströme kurz vor der Mischung im Reaktor über drallerzeugende Einbauten geleitet werden.

2. Verfahren nach Anspruch 1, **dadurch gekennzeichnet, dass** die Ausgangsstoffe verdünnt mit einem Inertgas und/oder mit Dämpfen eines inerten Lösungsmittels dem Reaktor zugeführt werden.

3. Verfahren nach Anspruch 1, **dadurch gekennzeichnet, dass** 1,6-Diaminohexan als Diamin der Formel (I) eingesetzt wird.

4. Verfahren nach Anspruch (I), **dadurch gekennzeichnet, dass** 1,6-Diisocyanatohexan (HDI) hergestellt wird.

5. Verfahren nach Anspruch 1, **dadurch gekennzeichnet, dass** 1,8-diamino-4-(aminomethyl)octan als Triamin der Formel (I) eingesetzt wird.

## Claims

1. Process for preparing (cyclo)aliphatic diisocyanates and triisocyanates of the general formula (I)
R-(NCO)ₙ (I),
where
R is a (cyclo)aliphatic hydrocarbon radical having up to 15 carbon atoms and
n is 2 or 3,
by phosgenation of the corresponding diamines or triamines of the general formula (II)
R-(NH₂)ₙ (II),
where
R and n have the meanings given for the formula (I),
in the gas phase, in which the gaseous (cyclo)aliphatic amines, if appropriate diluted with an inert gas or with the vapour of an inert solvent, and phosgene are heated separately to temperatures of from 200°C to 600°C and reacted continuously in a reaction space without moving parts with constriction of the walls in the region of the reaction zone, **characterized in that** a reactor whose cross-sectional area widens at an angle of 80 to 90° and in which the ratio of the cross-sectional areas of the reaction space after and before the widening is from 3:1 to 7:1 is used and the feed streams are passed over internals which generate rotational motion shortly before mixing in the reactor.

2. Process according to Claim 1, **characterized in that** the starting materials are diluted with an inert gas and/or with vapour of an inert solvent before being fed into the reactor.

3. Process according to Claim 1, **characterized in that** 1,6-diaminohexane is used as diamine of the formula (I).

4. Process according to Claim 1, **characterized in that** 1,6-diisocyanatohexane (HDI) is prepared.

5. Process according to Claim 1, **characterized in that** 1,8-diamino-4-(aminomethyl)octane is used as triamine of the formula (I).

## Revendications

1. Procédé de préparation en phase gazeuse de composés diisocyanates et triisocyanates (cyclo)aliphatiques de la formule générale (I)
R - (NCO)ₙ (I),
dans laquelle
R représente un radical hydrocarbure (cyclo)aliphatique comportant jusqu'à 15 atomes de carbone et
n équivaut au nombre 2 ou 3,
par phosgénation des diamines ou des triamines correspondantes de la formule générale (II)
R - (NH₂)ₙ (II),
dans laquelle
R et n possèdent la signification mentionnée pour la formule (I),
les amines (cyclo)aliphatiques sous la forme de vapeur, le cas échéant diluées avec un gaz inerte ou avec des vapeurs d'un solvant inerte, et le phosgène étant chauffés séparément à des températures de 200 à 600 °C, et mis en réaction continuellement dans un espace réactionnel dépourvu d'éléments mobiles et présentant des rétrécissements des parois au niveau de la zone réactionnelle, **caractérisé en ce que** l'on utilise un réacteur dont l'aire de la section transversale s'élargit sous un angle de 80 à 90° et dans lequel le rapport entre les aires de la section transversale de l'espace réactionnel après et avant l'élargissement est compris entre 3:1 et 7:1 et les flux d'éduits de réactifs étant acheminés peu avant le mélangeage dans le réacteur, via des chicanes générant des tourbillons.

2. Procédé selon la revendication 1, **caractérisé en ce que** les matières de départ sont acheminées au réacteur en étant diluées avec un gaz inerte et/ou des vapeurs d'un solvant inerte.

3. Procédé selon la revendication 1, **caractérisé en ce que** du 1,6-diaminohexane est utilisé comme diamine de la formule (I).

4. Procédé selon la revendication 1, **caractérisé en ce que** du 1 ,6-diisocyanatohexane (HDI) est préparé.

5. Procédé selon la revendication 1, **caractérisé en ce que** du 1,8-diamino-4-(aminométhyl)octane est utilisé comme triamine de la formule (I)
